# EUROPEAN PATENT SPECIFICATION

(11) **EP 0 358 880 B1**
(45) Date of publication and mention of the grant of the patent: **19.01.1994**
(21) Application number: 89112859.7
(22) Date of filing: 13.07.1989
(51) Int. Cl.: A61K 7/48, A61K 7/42, C07C 333/04

(54) **Use of retinoids**
Verwendung von Retinoiden
Utilisation de rétinoides

(30) Priority: 14.07.1988 US 219550
(43) Date of publication of application: 21.03.1990
(73) Proprietor: F. HOFFMANN-LA ROCHE AG, 4002 Basel (CH)
(72) Inventor: Bryce, Graeme Findlay, Upper Montclair, N.J. 07043 (US); Shapiro, Stanley Seymour, N.J. 07039 (US)
(74) Representative: Lederer, Franz, Dr.

(56) References cited:
- EP-A- 0 058 370

## Description

The skin, particularly in humans, contains an elaborate network of elastin fibers which are responsible for maintaining its elastic properties. With excessive exposure to sunlight the elastic fiber system becomes hyperplastic, disorganized and ultimately disrupted. This is known as actinic elastosis and is the principal cause of wrinkling, discoloration and laxity of the skin in the exposed areas of the body. The skin can repair itself to some extent but it is nevertheless desirable to have an agent which can accelerate the repair of this prematurely aged skin.

The UVB irradiated hairless mouse has been found to be a convenient model for actinic elastosis in the skin. (Kligman et al. J. Invest. Dermatol. 78:181 (1982). It has been shown by Johnston et al. in J. Invest. Dermatol. 82:587 (1984) that irradiation with low levels of UVB which simulate realistic solar exposure leads to a significant increase in skin elastin as measured by desmosine content. The amount of this amino acid, which is isolated from acid hydrolysis of elastin, is proportional to the elastin present in the skin. (Uitto et al., Lab. Invest. 49:1216 (1973). Treatment of irradiated mice with topical retinoic acid has been shown to normalize the histological features of the skin in which the previously elastotic dermis has the appearance of unirradiated tissue (Kligman et al., Conn. Tissue Res. 12:139 (1984), Kligman U.S. Patent No. 4,603,146 July 1986). Therefore this model can be used to determine the efficacy of compounds in the repair of sun damaged skin.

In accordance with this invention, it has been found that compounds of formula 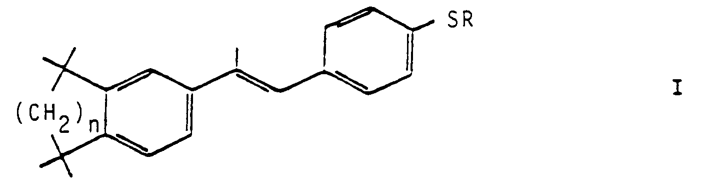
wherein n represents 1 or 2; and R represents mono- or di(C₁₋₄-alkyl)-carbamoyl
applied topically to the skin of a patient reverses the conditions associated with photodamage. Hence, by the topical application of compounds of formula I to the skin of patients which has been damaged through sun exposure, the effects of wrinkling, elastosis and premature aging can be reversed leading to an improvement in the appearance of the skin.

Through the topical administration of the compounds of the formula I, the acceleration of repair of dermal damage is accomplished so as to provide the skin with a smoother and younger appearance.

Alkyl groups can be straight-chain or branched-chain. Preferred C₁₋₄-alkyl groups are methyl, ethyl and isopropyl.

In one aspect, the invention relates to compounds of the formula I as defined above for use as a pharmaceutical agent.

An especially preferred compound of formula I is S-[p-[-2-(5,6,7,8-tetrahydro-5,5,,8-tetramethyl-2-naphthyl)propenyl]phenyl]dimethylthiocarbamate.

Compounds of formula I wherein R is di(lower alkyl)carbamoyl are known as intermediates from EP-A-0 058 370.

The compounds of formula I can be prepared by reacting a compound of formula 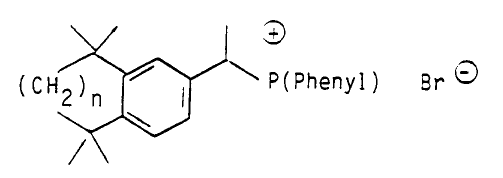
with a compound of formula 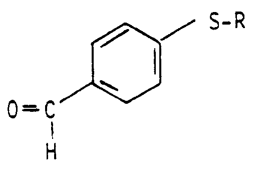
where R is mono- or di-C₁₋₄-alkylcarbamoyl.

The present invention is further concerned with pharmaceutical compositions containing as the active ingredient a compound of the formula I as defined above and to the manufacture of such compositions. The invention also relates to the use of compounds of formula I as defined above for the manufacture of pharmaceutical compositions for the topical treatment of skin conditions produced by photodamage.

The compounds of the formula I when applied topically to the skin, reverse the conditions associated with photodamage so as to moderate and retard the damage to the skin caused by sun exposure. The damage caused by sun exposure may include premature aging, elastosis and wrinkling. This damage is more pronounced in older patients. By applying the compounds of formula I topically to the skin in an amount effective to reverse the conditions associated with photodamage, the acceleration of skin repair is accomplished to enhance the skin with a smoother and younger appearance. The compounds of formula I should be applied to that portion or area of the skin which is affected by photodamage or in which treatment is desired. The use of the compounds of formula I in accordance with this invention can provide the effects of anti-aging and anti-wrinkling, as well as enhance the repair of sun damaged skin.

A compound of formula I, or a combination of compounds of formula I can be applied in accordance with this invention to human skin in conventional topical compositions. These compositions can be utilized to apply compounds of formula I to the skin of the body, particularly the face, legs, arms and hands. The preferred method of application of compounds of formula I topically to produce the best effects should start where a patient is between 30 and 55 years of age, when elastosis begins to appear and becomes more pronounced. Thereafter, this composition can be continuously applied to patients to reduce the effects and injury associated with sun exposure. Generally, it is preferred to begin the treatment when the patient reaches approximately 30 years of age and to continue the treatment throughout his life, in order that the effects of elastosis be reduced and to prevent any further progression of photodamage.

The compounds of formula I can be administered in accordance with this invention in any conventional suitable topical preparation, i.e. in combination with any suitable conventional carrier useful for topical administration. Therefore, compounds of formula I can be administered in accordance with this invention in any suitable topical composition such as a cream, ointment, soap, solution, lotion, emulsion, shampoo, etc. Generally, for most efficacious results, these topical compositions contain from about 0.00001% to about 1.0% by weight of the total composition of a compound of formula I, with amounts of from about 0.0001% to about 0.1% by weight of the composition being especially preferred. If desired, higher concentrations may be utilized depending upon the nature and extent of elastosis.

In formulating these compositions, any conventional non-toxic, dermatologically acceptable base or carrier in which a compound of formula I is stable can be utilized. The preferred compositions for use in this invention are the conventionally cosmetic compositions which can contain a cosmetically active ingredient which is topically administered to human skin to provide a cosmetic effect. Among the conventional cosmetically active materials which can be utilized in this composition are included: sunscreens, penetration enhancers, moisturizers, surfactants, emollients, colorants, conditioners, bacteriocides, astringents, detergents, etc.

The topical compositions of this invention can, if desired contain suitable sunscreen agents. Any conventional sunscreen agent can be utilized in formulating the formulations containing compounds of formula I which can be utilized in accordance with this invention.

These topical compositions which contain compounds of formula I can contain any of the conventional excipients and additives commonly used in preparing topical compositions. Among the conventional additives or excipients, which can be utilized in preparing these cosmetic compositions in accordance with this invention are preservatives, thickeners, perfumes and the like. In addition, the conventional antioxidants, such as butylated hydroxyanisoles (BHA), ascorbyl palmitate, propyl gallate, citric acid butylated hydroxy toluene (BHT), ethoxyquin, tocopherol, and the like can be incorporated into these compositions. These topical compositions can contain conventional acceptable carriers for topical applications which are generally utilized in these compositions. These compositions may contain thickening agents, humectants, emulsifying agents and viscosity stabilizers, such as those generally utilized. In addition, these compositions can contain flavoring agents, colorants, and perfume which are conventional in preparing cosmetic compositions.

The topical compositions containing compounds of formula I can be applied to the skin and should be preferably applied to the skin at least once a day for at least 2 or 3 times a week. For obtaining the reversal of the elastosis so as to impart to the skin a smooth and younger appearance the topical compositions should be preferably applied for a period of at least five months. After that compositions which contain compounds of formula I should be applied continually to maintain the effect of younger and smoother skin. These preparations can be applied according to the need of the patient as determined by the prescribing physician. In any event, the particular regimen for application of this composition to a patient will typically depend on the age, weight and skin condition of the individual.

The invention is further illustrated in the following examples. These examples are for illustration and are not limitative of the claimed invention.

### Example 1

99 g of [1-(5,6,7,8-Tetrahydro-5,5,8,8-tetramethyl-2-naphthyl)ethyl]-triphenylphosphonium bromide are heated at reflux for 40 hours with 25.2 g of S-(p-formylphenyl)dimethylthiocarbamate in 1 l of butylene oxide. The mixture is cooled, poured into 1 l of methanol/water (6:4) and extracted three times with hexane. The organic phase is washed with water, dried over sodium sulphate and evaporated. The crude product is filtered over silica gel using hexane/ethyl acetate (4:1) for the elution and recrystallized from ethyl acetate/hexane. There are obtained 39 g of S-[p-[2-(5,6,7,8-Tetrahydro-5,5,8,8-tetramethyl-2-naphthyl)propenyl]phenyl]dimethylthiocarbamate in the form of colourless crystals, melting point 107-109°C.

The S-(p-formylphenyl)dimethylthiocarbamate (melting point 78-80°C) used as the starting material can be prepared from p-hydroxybenzaldehyde via O-(p-formylphenyl)dimethylthiocarbamate (melting point 94-96°C) according to the procedure described by M.S. Newmann and H.A. Karnes in J. Org. Chem. 31, 3980 (1966).

### Example 2

### Repair of UVB-Induced Dermal Damage in the Hairless Mouse by Compounds of Formula I

Hairless mice (HRS/J strain, Jackson Labs, 5-7 weeks old at the start of the experiments) were irradiated three times per week with a bank of 8 Westinghouse® Sunlamps (FS40) placed about 20cm above the animals. The radiation dose was controlled by an International Light Model IL844A Phototherapy Exposure Control and a detector. The UVB dosing schedule was such that individual doses, seldom exceeding 0.06J/cm², caused minimal erythema but no burning or scarring. There was significant elastosis, detected by histology, after a total dose of about 3.5J/cm²: this was confirmed in measurements of elastin in whole skin by means of a radioimmunoassay for desmosine. Desmosine is found in elastin hydrolysates and is derived from crosslinks in the elastin molecule; it is a reliable index of total elastin. Typically, desmosine increased by about 2-3 fold after 3.5J/cm² of UVB irradiation. To effect repair of the dermal damage, the UVB irradiation was discontinued and animals were treated three times per week with various concentrations of the compounds of formula I dissolved in acetone. Solutions were made up freshly every week at concentrations such that the dose was delivered in 100µl acetone and applied topically to an area of about 10 cm² on the back of the animal with a plastic pipette; a control group treated with acetone alone was also included.

After 10 weeks of treatment the animals were sacrificed, skin samples were taken and processed by standard methods. A six µm [micron] section from each animal was stained for elastin with Luna's stain and the degree of repair measured quantitatively. In this model, repair is defined by the appearance of a normalized dermis extending from the epidermis down to the layer of compressed elastin. The extent of repair was reflected by the width of this zone. In these studies, the area of the zone on a standard length of histological section was measured by an image analyzer and the results are given as total area in mm² per twenty microscopic fields. Data was analyzed by Student's t-test. The results are given in Table I. In Table I, and Table II each group dosed at a particular concentration of compound of formula I contained six to ten animals.

**Table I**

| Group | Repair Zone, mm² |
|---|---|
| Control | 0.007 +/- 0.003 |
| 0.1 µg of Compound A | 0.005 +/- 0.001 |
| 0.5 µg | 0.006 +/- 0.003 |
| 2.0 µg | 0.021 +/- 0.003* |
| 10.0 µg | 0.015 +/- 0.004 |
| 50.0 µg | 0.025 +/- 0.007* |

| | |
|---|---|
| * P < 0.05 vs Control | |

Throughout the specification,
Compound A is S-[p-(5,6,7,8-tetrahydro-5,5,8,8-tetramethyl-2-naphthylpropenyl]phenyl]dimethylthiocarbamate.

### Example 3

### Effect of Compounds of Formula I on the Wrinkles Produced in Hairless Mice by UVB-Irradiation

Doses of UVB-irradiation sufficient to induce dermal damage in hairless mice were found to cause the appearance of wrinkles on the exposed skin. One skin replica for each animal was taken of these areas using a liquid dental impression material (SILFLO® - Flexico Developments Ltd., England). Wrinkles appeared in these impressions as ridges which cast a shadow when illuminated with low angle light. A characteristic of the wrinkling pattern was the occurrence of the ridges in a regularly-spaced array about 2-mm apart. The extent of wrinkling was visually assessed using this line pattern and assigned a value (Wrinkle Index) of zero to 4 with zero representing complete effacement of wrinkling and 4 representing the maximum degree of wrinkling. It was observed that compounds of formula I caused a dose-dependent effacement of the wrinkles with ED₅₀ values in the microgram range. The results are shown in the following Table.

| Group | Wrinkle Index |
|---|---|
| Control | 1.9 +/- 0.3 |
| 0.1 µg of Compound A | 1.4 +/- 0.5 |
| 0.5 µg | 1.0 +/- 0.3* |
| 2.0 µg | 0.8 +/- 0.2** |
| 10.0 µg | 0.3 +/- 0.1*** |
| 50.0 µg | 0.3 +/- 0.1*** |

| | |
|---|---|
| * P < 0.05, | |
| ** P < 0.01, | |
| *** P < 0.001 vs Control | |

Creams and gels containing ingredients within the proportions set forth in Examples 3 through 7 below can be formulated by conventional means.

### EXAMPLE 4

| CREAM | |
|---|---|
| | % w/w |
| Compound of formula I | 0.00001-1.3 |
| Cetyl Alcohol | 1.5 |
| Stearyl Alcohol | 2.5 |
| Span® 60 (Sorbitan Stearate) | 2.0 |
| Mineral Oil | 2.0 |
| Arlacel® 165 (Glyceryl/PEG 100 Stearate) | 4.0 |
| Tween® 60 (Polysorbate 80) | 1.0 |
| Miglyol® 818 (Caprylic/Capric/Linoleic triglyceride) | 5.0 |
| Sorbitol Solution | 4.0 |
| Disodium Edetate | 0.1 |
| BHA (Butylated Hydroxyanisole) | 0.05 |
| Methylparaben | 0.18 |
| Propylparaben | 0.05 |
| Water q.s. | 100.00 |

### EXAMPLE 5

| CREAM | |
|---|---|
| | % w/w |
| Compound of formula I | 0.00001-1.0 |
| Cetyl Alcohol | 5.25-8.75 |
| Arlacel® 165 (Glyceryl/PEG 100 Stearate) | 3.75-6.25 |
| Miglyol® 818 (Caprylic/Capric/Linoleic triglyceride) | 11.25-18.75 |
| Sorbitol Solution | 3.75-6.25 |
| Disodium Edetate | .075-0.125 |
| Carbopol® 934P (Carbomer 934P) | 0.15-0.25 |
| BHA (Butylated Hydroxyanisole) | 0.0375-0.0625 |
| Methylparaben | 0.135-0.225 |
| Propylparaben | 0.0375-0.0625 |
| Sodium Hydroxide (10% solution) | 0.15-0.25 |
| Distilled Water, q.s. | 100.00 |

### EXAMPLE 6

| CREAM | |
|---|---|
| | % w/w |
| Compound of formula I | 0.00001-1.0 |
| Cutina® MD (Glyceryl Stearate) | 4.5-7.5 |
| Ceteareth®-12 | 3.0-5.0 |
| Cetyl Alcohol | 3.0-5.0 |
| Generol® 122E-10 (Ethoxylated Soya Sterol) | 2.25-3.75 |
| Cetiol® LC (Oleic Acid Decyl Ester) | 7.5-12.5 |
| BHA (Butylated Hydroxyanisole) | 0.0375-0.0625 |
| Sorbitol Solution | 3.75-6.25 |
| Disodium Edetate | 0.075-0.125 |
| Methylparaben | 0.135-0.225 |
| Propylparaben | 0.0375-0.0625 |
| Distilled Water, q.s. | 100.00 |

### EXAMPLE 7

| CREAM | |
|---|---|
| | % w/w |
| Compound of formula I | 0.00001-1.0 |
| Arlatone® 983 (PEG 30/Glyceryl Stearate) | 7.0 |
| Cetyl Alcohol | 1.0 |
| Stearic Acid | 4.0 |
| Neobee® Oil (Medium chain-length triglyceride) | 17.0 |
| Propylene Glycol | 5.0 |
| 2-phenoxyethanol | 0.5 |
| Distilled Water, q.s | 100.00 |

### EXAMPLE 8

| GEL | |
|---|---|
| | % w/w |
| Compound of formula I | 0.00001-1.0 |
| Pluronic® L 101 (Poloxamer 331) | 10.00 |
| Aerosil® 200 (Silica) | 8.00 |
| PCL Liquid (Fatty Acid Esters) | 15.00 |
| Cetiol® V (Decyl Oleate) | 20.00 |
| Neobee® Oil (Medium chain-length triglyceride) | 15.00 |
| Euhanol® G (Octyldodecanol), q.s. | 100.00 |

It is understood that the proportions of excipients in creams of Examples 3 and 6, and the gels of Example 7 can be varied, if desired, to change the physical properties of the resulting creams and gels.

## Claims (Claims for the following Contracting State(s): AT, BE, CH, DE, FR, GB, IT, LI, LU, NL, SE)

1. A compound of the formula 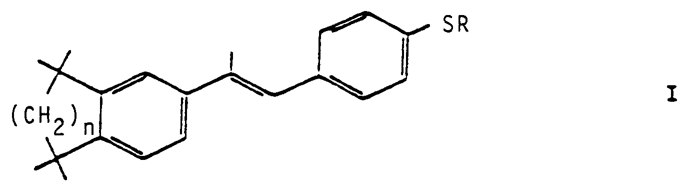 wherein R is mono- or di-(C₁₋₄-alkyl)carbamoyl and n is 1 or 2;
for use as a pharmaceutical agent.

2. A compound according to claim 1 which is S-[p-[2-(5,6,7,8-tetrahydro-5,5,8,8-tetramethyl-2-naphthyl)propenyl]phenyl]dimethylthiocarbamate.

3. A pharmaceutical composition for topical application containing, additionally to any conventional non-toxic dermatologically acceptable base or carrier and/or cosmetically active ingredients and/or excipients and/or additives, as the active ingredient a compound of formula I as defined in Claim 1.

4. A pharmaceutical composition according to claim 3 wherein the active ingredient is S-[p-[2-(5,6,7,8-tetrahydro-5,5,8,8-tetramethyl-2-naphthyl)propenyl]phenyl]dimethylthiocarbamate.

5. A pharmaceutical composition according to claim 3 or 4 containing a compound of formula I in amount of from 0.0001 to 0.1% by weight of the composition.

6. The composition of claim 5, which contains a cosmetically active ingredient.

7. The composition of claim 6 wherein said cosmetically active ingredient is a sunscreen.

8. The composition of any one of claims 3 to 7, which is a gel, cream or ointment.

9. The use of a compound of formula I as defined in claim 1 for the manufacture of pharmaceutical preparations for the topical treatment of skin disorders produced by photodamage.

10. The use according to claim 9 wherein the compound of formula I is S-[p-[2-(5,6,7,8-tetrahydro-5,5,8,8-tetramethyl-2-naphthyl)propenyl]phenyl]dimethylthiocarbamate.

11. The use according to claims 9 or 10 wherein the skin disorder is wrinkling, elastosis and premature aging.

## Claims (Claims for the following Contracting State(s): ES)

1. The use of a compound of the formula 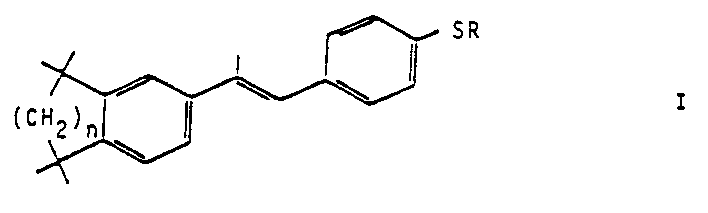 wherein R is mono- or di-(C₁₋₄ alkyl)carbamoyl and n is 1 or 2;
for the manufacture of pharmaceutical preparations for the topical treatment of skin disorders produced by photodamage.

2. The use according to claim 1 wherein the compound of formula I is S-[p-[2-(5,6,7,8-tetrahydro-5,5,8,8-tetramethyl-2-naphthyl)propenyl]phenyl]dimethylthiocarbamate.

3. The use according to claims 1 or 2 wherein the skin disorder is wrinkling, elastosis and premature aging.

## Claims (Claims for the following Contracting State(s): GR)

1. Process for the manufacture of pharmaceutical compositions for topical application which comprises mixing a compound of the formula 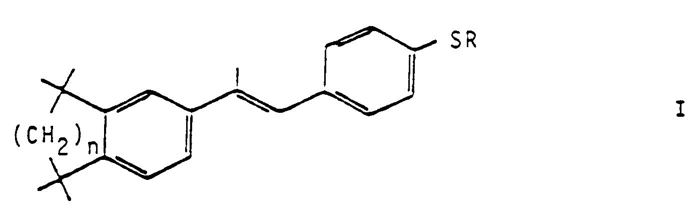 wherein R is mono- or di-(C₁₋₄-alkyl)carbamoyl and n is 1 or 2;
with an appropriate conventional pharmaceutical carrier.

2. A process as in claim 1 wherein the compound of formula I is S-[p-[2-(5,6,7,8-tetrahydro-5,5,8,8-tetramethyl-2-naphthyl)propenyl]phenyl]dimethylthiocarbamate.

3. A process as in claim 1 or 2 wherein the compound of formula I is present in an amount of from 0.0001 to 0.1% by weight of the composition.

4. A process as in any one of claims 1-3 wherein a cosmetically active ingredient is added.

5. A process as in claim 4 wherein said cosmetically active ingredient is a sunscreen.

6. A process as in any one of claims 1-5 wherein said composition is a gel, cream or ointment.

7. The use of a compound of formula I as defined in claim 1 for the manufacture of pharmaceutical preparations for the topical treatment of skin disorders produced by photodamage.

8. The use according to claim 7 wherein the compound of formula I is S-[p-[2-(5,6,7,8-tetrahydro-5,5,8,8-tetramethyl-2-naphthyl)propenyl]phenyl]dimethylthiocarbamate.

9. The use according to claims 7 or 8 wherein the skin disorder is wrinkling, elastosis and premature aging.

## Patentansprüche (Patentansprüche für folgende(n) Vertragsstaat(en): AT, BE, CH, DE, FR, GB, IT, LI, LU, NL, SE)

1. Eine Verbindung der Formel 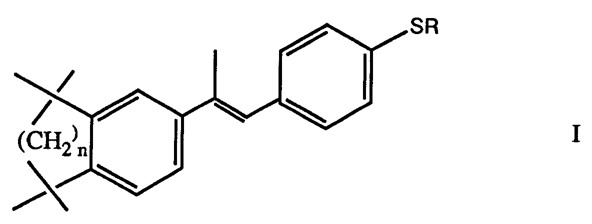 worin R mono- oder di-(C₁₋₄-Alkyl)carbamoyl und n 1 oder 2 ist, zur Verwendung als pharmazeutischer Wirkstoff.

2. Eine Verbindung gemäss Anspruch 1, die S-[p-[2-(5,6,7,8-Tetrahydro-5,5,8,8-tetramethyl-2-naphthyl)propenyl]pheny]dimethylthiocarbamat ist.

3. Ein pharmazeutisches Präparat zur topischen Anwendung, enthaltend als aktiven Inhaltsstoff einer Verbindung der Formel I gemäss Definition in Anspruch 1 zusätzlich zu konventionellen, nicht-toxischen, dermatologisch anwendbaren Basen oder Trägern und/oder kosmetisch aktiven Inhaltsstoffen und/oder Excipientien und/oder Zusatzstoffen.

4. Ein pharmazeutisches Präparat gemäss Anspruch 3, worin der aktive Wirkstoff S-[p-[2-(5,6,7,8-Tetrahydro-5,5,8,8-tetramethyl-2-naphthyl)propenyl]phenyl]dimethylthiocarbamat ist.

5. Ein pharmazeutisches Präparat gemäss Anspruch 3 oder 4 enthaltend eine Verbindung der Formel I in einer Menge von 0,0001 bis 0,1 Gew.%.

6. Das Präparat gemäss Anspruch 5 das einen kosmetisch wirksamen Inhaltsstoff enthält.

7. Das Präparat gemäss Anspruch 6, worin der kosmetische aktive Inhaltsstoff ein Lichtschutzmittel ist.

8. Das Präparat gemäss einem der Ansprüche 3-7 das ein Gel, eine Crème oder eine Salbe ist.

9. Die Verwendung einer Verbindung der Formel I gemäss Definition in Anspruch 1 zur Herstellung eines pharmazeutischen Präparates zur topischen Behandlung von Hauterkrankungen, die durch Lichtschäden verursacht wurden.

10. Die Verwendung gemäss Anspruch 9, worin die Verbindung der Formel I S-[p-[2-(5,6,7,8-Tetrahydro-5,5,8,8-tetramethyl-2-naphthyl)propenyl]phenyl]dimethylthiocarbamat ist.

11. Die Verwendung gemäss den Ansprüchen 9 oder 10, worin die Hauterkrankung Faltigwerden, Elastose und verfrühtes Altern ist.

## Patentansprüche (Patentansprüche für folgende(n) Vertragsstaat(en): ES)

1. Verwendung einer Verbindung der Formel 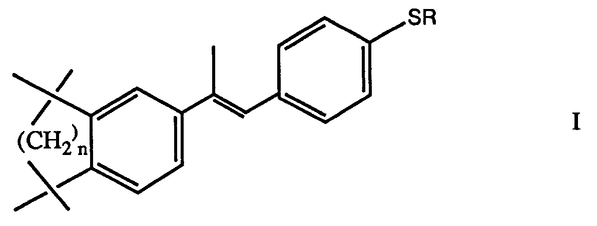 worin R mono- oder di-(C₁₋₄-Alkyl)carbamoyl und n 1 oder 2 ist, zur Herstellung eines pharmazeutischen Präparates zur topischen Behandlung von Hauterkrankungen, die durch Lichtschäden verursacht wurden.

2. Die Verwendung gemäss Anspruch 1, worin die Verbindung der Formel I S-[p-[2-(5,6,7,8-Tetrahydro-5,5,8,8-tetramethyl-2-naphthyl)propenyl]phenyl]dimethylthiocarbamat ist.

3. Die Verwendung gemäss den Ansprüchen 1 oder 2, worin die Hauterkrankung Faltigwerden, Elastose und verfrühtes Altern ist.

## Patentansprüche (Patentansprüche für folgende(n) Vertragsstaat(en): GR)

1. Verfahren zur Herstellung pharmazeutischer Präparate zur topischen Anwendung durch Vermischen einer Verbindung der Formel 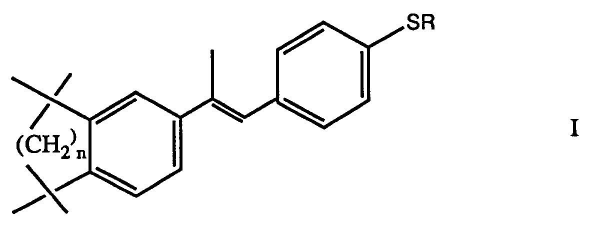 worin R mono- oder di-(C₁₋₄-Alkyl)carbamoyl und n 1 oder 2 ist, mit einem geeigneten konventionellen pharmazeutischen Träger.

2. Verfahren gemäss Anspruch 1, worin der aktive Wirkstoff S-[p-[2-(5,6,7,8-Tetrahydro-5,5,8,8-tetramethyl-2-naphthyl)propenyl]phenyl]dimethylthiocarbamat ist.

3. Verfahren gemäss Anspruch 1 oder 2 wobei die Verbindung der Formel I in einer Menge von 0,0001 bis 0,1 Gew.% des Präparats anwesend ist.

4. Verfahren gemäss den Ansprüchen 1-3, wobei ein kosmetisch wirksamer Inhaltsstoff zugesetzt wird.

5. Verfahren gemäss Anspruch 4, worin der kosmetische aktive Inhaltsstoff ein Lichtschutzmittel ist.

6. Verfahren gemäss einem der Ansprüche 1-5 , wobei das Präparat ein Gel, eine Crème oder eine Salbe ist.

7. Die Verwendung einer Verbindung der Formel I gemäss Definition in Anspruch 1 zur Herstellung eines pharmazeutischen Präparates zur topischen Behandlung von Hauterkrankungen, die durch Lichtschäden verursacht wurden.

8. Die Verwendung gemäss Anspruch 7, worin die Verbindung der Formel I S-[p-[2-(5,6,7,8-Tetrahydro-5,5,8,8-tetramethyl-2-naphthyl)propenyl]phenyl]dimethylthiocarbamat ist.

9. Die Verwendung gemäss den Ansprüchen 7 oder 8, worin die Hauterkrankung Faltigwerden, Elastose und verfrühtes Altern ist.

## Revendications (Revendications pour l'(les) Etat(s) contractant(s) suivant(s): AT, BE, CH, DE, FR, GB, IT, LI, LU, NL, SE)

1. Composé de formule : 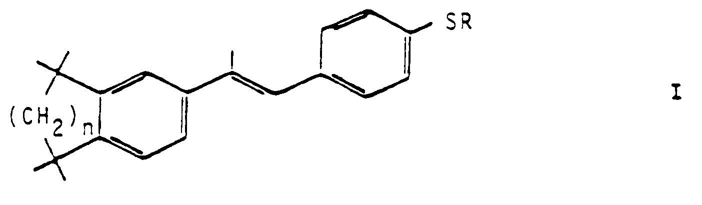 dans laquelle R est un groupe mono- ou di-(alkyl en C₁-C₄)carbamoyle et n est égal à 1 ou 2;
utilisable comme agent pharmaceutique.

2. Composé selon la revendication 1, qui est le thiocarbamate de S-[p-[2-(5,6,7,8-tétrahydro-5,5,8,8-tétraméthyl-2-naphtyl)propényl]phényl]diméthyle.

3. Composition pharmaceutique pour application en topique, contenant comme ingrédient actif, en plus d'une base ou d'un véhicule non toxique classique acceptable en dermatologie et/ou d'ingrédients et/ou d'excipients et/ou d'additifs actifs en cosmétique, un composé de formule I définie dans la revendication 1.

4. Composition pharmaceutique selon la revendication 3, dans laquelle l'ingrédient actif est le thiocarbamate de S-[p-[2-(5,6,7,8-tétrahydro-5,5,8,8-tétraméthyl-2-naphtyl)propényl]phényl]diméthyle.

5. Composition pharmaceutique selon la revendication 3 ou 4, contenant un composé de formule I, en une quantité de 0,0001 à 0,1% du poids de la composition.

6. Composition selon la revendication 5, qui contient un ingrédient actif en cosmétique.

7. Composition selon la revendication 6, dans laquelle ledit ingrédient actif en cosmétique est un écran anti-solaire.

8. Composition selon l'une quelconque des revendications 3 à 7, qui est un gel, une crème ou une pommade.

9. Utilisation d'un composé de formule I telle que définie dans la revendication 1, pour fabriquer des préparations pharmaceutiques pour le traitement en topique d'affections cutanées produites par des lésions photoniques.

10. Utilisation selon la revendication 9, dans laquelle le composé de formule I est le thiocarbamate de S-[p-(2-(5,6,7,8-tétrahydro-5,5,8,8-tétraméthyl-2-naphtyl)propényl]phényl]diméthyle.

11. Utilisation selon la revendication 9 ou 10, dans laquelle l'affection cutanée est un ridage, une élastose et un vieillissement prématuré.

## Revendications (Revendications pour l'(les) Etat(s) contractant(s) suivant(s): ES)

1. Utilisation d'un composé de formule : 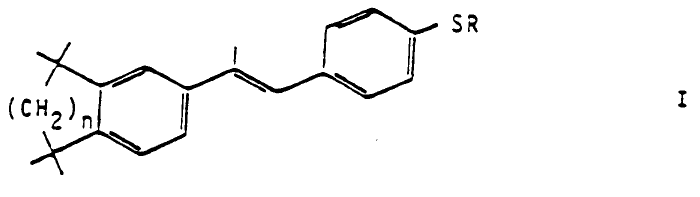 dans laquelle R est un groupe mono- ou di-(alkyl en C₁-C₄)carbamoyle et n est égal à 1 ou 2;
pour fabriquer des préparations pharmaceutiques pour le traitement en topique d'affections cutanées produites par des lésions photoniques.

2. Utilisation selon la revendication 1, dans laquelle le composé de formule I est le thiocarbamate de S-[p-[2-(5,6,7,8-tétrahydro-5,5,8,8-tétraméthyl-2-naphtyl)propényl]phényl]diméthyle.

3. Utilisation selon la revendication 1 ou 2, dans laquelle l'affection cutanée est un ridage, une élastose et un vieillissement prématuré.

## Revendications (Revendications pour l'(les) Etat(s) contractant(s) suivant(s): GR)

1. Procédé de fabrication de compositions pharmaceutiques pour application en topique, qui comprend le mélange d'un composé de formule 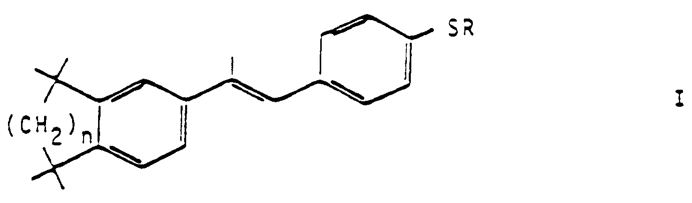 dans laquelle R est un groupe mono- ou di-(alkyl en C₁-C₄)carbamoyle et n est égal à 1 ou 2;
avec un véhicule pharmaceutique classique approprié.

2. Procédé selon la revendication 1, dans lequel le composé de formule I est le thiocarbamate de S-[p-[2-(5,6,7,8-tétrahydro-5,5,8,8-tétraméthyl-2-naphtyl)propényl]phényl]diméthyle.

3. Procédé selon la revendication 1 ou 2, dans lequel le composé de formule I est présent en une quantité de 0,0001 à 0,1% en poids de la composition.

4. Procédé selon l'une quelconque des revendications 1-3, dans lequel on ajoute un ingrédient actif en cosmétique.

5. Procédé selon la revendication 4, dans lequel ledit ingrédient actif en cosmétique est un écran anti-solaire.

6. Procédé selon l'une quelconque des revendications 1 à 5, dans lequel ladite composition est un gel, une crème ou une pommade.

7. Utilisation d'un composé de formule I telle que définie dans la revendication 1, pour fabriquer des préparations pharmaceutiques pour le traitement en topique d'affections cutanées produites par des lésions photoniques.

8. Utilisation selon la revendication 7, dans laquelle le composé de formule I est le thiocarbamate de S-[p-[2-(5,6,7,8-tétrahydro-5,5,8,8-tétraméthyl-2-naphtyl)propényl]phényl]diméthyle.

9. Utilisation selon la revendication 7 ou 8, dans laquelle l'affection cutanée est un ridage, une élastose et un vieillissement prématuré.
